(19) Europäisches Patentamt
European Patent Office
Office européen des brevets

(11) **EP 4 223 323 A1**

(12) **EUROPEAN PATENT APPLICATION**
published in accordance with Art. 153(4) EPC

(43) Date of publication:
**09.08.2023 Bulletin 2023/32**

(21) Application number: **21875898.5**

(22) Date of filing: **01.10.2021**

(51) International Patent Classification (IPC):
**A61L 2/20** (2006.01)      **A61L 9/01** (2006.01)
**A61L 9/015** (2006.01)      **A61L 101/36** (2006.01)

(52) Cooperative Patent Classification (CPC):
**A61L 2/20; A61L 9/01; A61L 9/015;** A61L 2101/00

(86) International application number:
**PCT/JP2021/036527**

(87) International publication number:
**WO 2022/071603 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2020   JP 2020167523**

(71) Applicant: **Nitta Corporation**
**Osaka-shi, Osaka 556-0022 (JP)**

(72) Inventors:
• **SHIGETA, Makoto**
**Yamatokooriyama-shi, Nara 639-1085 (JP)**
• **IKEDA, Takuji**
**Yamatokooriyama-shi, Nara 639-1085 (JP)**

(74) Representative: **Hasegawa, Kan**
**Patentanwaltskanzlei Hasegawa**
**Untere Hauptstraße 56**
**85354 Freising (DE)**

(54) **DECONTAMINATION METHOD**

(57)      A decontamination method is a decontamination method for decontaminating the inside of a decontamination target by removing at least one of microorganisms and viruses present in the decontamination target. This decontamination method includes a step of releasing vapor containing peracetic acid into the decontamination target, without releasing mist containing peracetic acid. An average humidity inside the decontamination target during decontamination is RH82% or more. A peracetic acid vapor load that is a value obtained by adding a concentration of peracetic acid in the vapor every minute from a start of decontamination to a completion of decontamination is 2000 ppm or more.

Fig. 1

EP 4 223 323 A1

## Description

Technical Field

The present invention relates to a decontamination method, and in particular to a decontamination method for decontaminating the inside of a decontamination target by removing at least one of microorganisms and viruses present inside the decontamination target.

Background Art

JP 2018-121826A (Patent Literature 1) discloses a decontamination device that decontaminates sterile manufacturing facilities etc. This decontamination device decontaminates a decontamination target space using dry fog containing peracetic acid (bio-decontamination).

Citation List

Patent Literature

Patent Literature 1: JP 2018-121826A

Summary of Invention

Technical Problem

The inventor(s) of the present invention considered decontaminating the inside of a decontamination target using vapor containing peracetic acid, instead of dry fog (mist) containing peracetic acid. Patent Literature 1 above does not disclose what method can be used to appropriately decontaminate the inside of a decontamination target when the inside of the decontamination target is to be decontaminated using vapor containing peracetic acid.

The present invention was made to resolve such issues, and aims to provide a decontamination method by which the inside of a decontamination target can be appropriately decontaminated using vapor containing peracetic acid.

Solution to Problem

The decontamination method according to the present invention is a decontamination method for decontaminating an inside of a decontamination target by removing at least one of a microorganism and a virus present in the decontamination target. This decontamination method includes releasing vapor containing peracetic acid into the decontamination target, without releasing mist containing peracetic acid. An average humidity inside the decontamination target during decontamination is RH82% or more. A peracetic acid vapor load that is a value obtained by adding a concentration of peracetic acid in the vapor every minute from a start of the decontamination to a completion of the decontamination is 2000 ppm or more.

The inventor(s) of the present invention found that, when the average humidity inside the decontamination target during decontamination is RH82% or more and the peracetic acid vapor load is 2000 ppm or more, the decontamination of the inside of the decontamination target succeeds. In the decontamination method according to the present invention, the average humidity inside the decontamination target during decontamination is RH82% or more and the peracetic acid vapor load is 2000 ppm or more. Thus, according to this decontamination method, it is possible to appropriately decontaminate the inside of the decontamination target.

In the decontamination method, the average humidity may be RH99% or less, and the peracetic acid vapor load may be 12000 ppm or less.

If the average humidity inside the decontamination target during decontamination exceeds RH99%, dew condensation may occur in the decontamination target. Dew condensation occurring in the decontamination target may cause corrosion of the inside of the decontamination target. Also, when the peracetic acid vapor load exceeds 12000 ppm, a corrodible metal part and the like inside the decontamination target tends to be corroded. Further, when the concentration of peracetic acid exceeds 100 ppm, a metal part inside the decontamination target or the like tends to be corroded. The inventor(s) of the present invention found these facts. In the decontamination method according to the present invention, the average humidity is RH99% or less, and the peracetic acid vapor load is 12000 ppm or less. Thus, according to this decontamination method, it is possible to suppress corrosion of the inside of the decontamination device.

In the decontamination method, the peracetic acid vapor load and the average humidity may further satisfy the following relationship.

$$y > -350x + 33250$$

x: the average humidity
y: the peracetic acid vapor load

The inventor(s) of the present invention found that decontamination will succeed when the peracetic acid vapor load and the average humidity satisfy the relationship. In the decontamination method according to the present invention, the peracetic acid vapor load and the average humidity satisfy the above relationship. Thus, according to this decontamination method, it is possible to appropriately decontaminate the inside of the decontamination target.

In the decontamination method, the vapor may be released for 50% or more of a time period from the start of the decontamination to the completion of the decontamination.

In the case of a method for releasing mist containing peracetic acid (also referred to as a "mist method" hereinafter), the mist floats in the space to be decontaminated, and thus dew condensation is likely to occur. In order to prevent dew condensation inside the decontamination target, the upper limit of the humidity for generating mist is set, and the mist is released intermittently. The upper limit of the humidity for generating mist is often RH80% to RH92%. In the case of the mist method, the time period during which mist is released is often less than 30% of the time period from the start of the decontamination to the completion of the decontamination.

On the other hand, in the case of the method for releasing vapor containing peracetic acid (also referred to as a "vaporization method" hereinafter), only vapor floats and no mist floats, and thus dew condensation is unlikely to occur.

In the decontamination method according to the present invention, it is possible to release vapor for a long time period of the time period from the start of the decontamination to the completion of the decontamination. The vapor can be released for 50% or more of the time period, for example. It is possible to release vapor for 100% of the time period from the start of the decontamination to the completion of the decontamination depending on a decontamination environment. Therefore, according to this decontamination method, because the vaporization method is adopted and the period of time in which the release of vapor is stopped is short, it is possible to inhibit peracetic acid vapor from decomposing in the decontamination target space and inhibit the concentration of peracetic acid from greatly decreasing.

In the decontamination method, the longest time period of the time period during which the vapor is continuously released may be 50% or more of the time period from the start of the decontamination to the completion of the decontamination.

According to this decontamination method, because the vaporization method is adopted and vapor is continuously released for a long time period, it is possible to inhibit the concentration of peracetic acid from greatly decreasing.

Advantageous Effects of Invention

According to the present invention, it is possible to provide a decontamination method by which the inside of the decontamination target can be appropriately decontaminated using vapor containing peracetic acid.

Brief Description of Drawings

FIG. 1 is a diagram showing a schematic configuration of a decontamination system.
FIG. 2 is a diagram showing a schematic configuration of a vapor generation unit.
FIG. 3 is a flowchart showing an example of a procedure of decontamination in a safety cabinet.
FIG. 4 is a diagram showing a schematic configuration of a vapor generation unit according to a first modification example.
FIG. 5 is a diagram showing a schematic configuration of a vapor generation unit according to a second modification example.
FIG. 6 is a diagram showing results in a case where a BI with $10^4$ was used.
FIG. 7 is a diagram showing results in a case where a BI with $10^6$ was used.

Description of Embodiments

Hereinafter, an embodiment of the present invention will be described in detail with reference to the drawings. Note that, in the drawings, identical or corresponding portions have been assigned the same reference numerals, and their explanation is not repeated.

1. Configuration of decontamination system

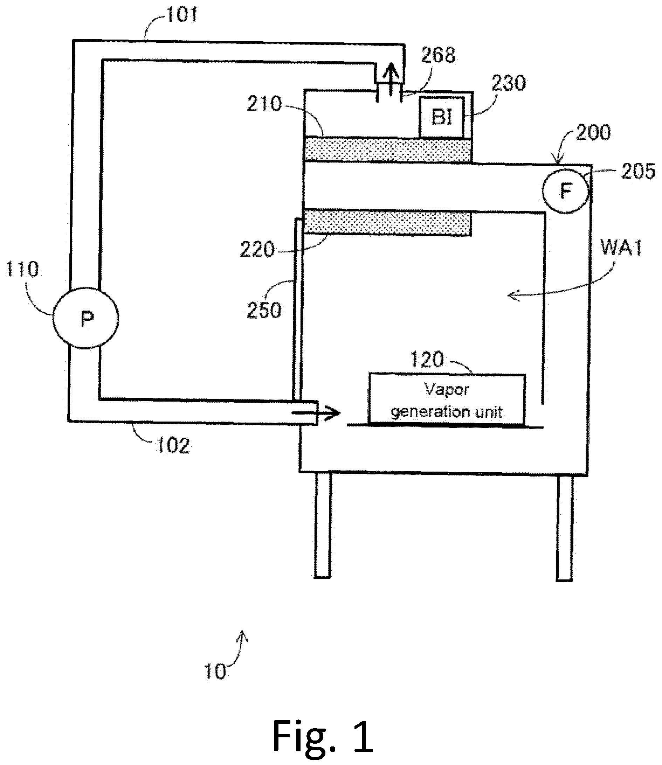

FIG. 1 is a diagram showing a schematic configuration of a decontamination system 10. As shown in FIG. 1, the decontamination system 10 includes a pump 110, a vapor generation unit 120, and a safety cabinet 200. In the decontamination system 10, the pump 110 is connected to the safety cabinet 200 via pipes 101 and 102. Although the vapor generation unit 120 is disposed in the safety cabinet 200, the vapor generation unit 120 may be disposed outside the safety cabinet 200, for example, on the paths of the pipes 101 and 102, for example. The vapor generation unit 120 is configured to generate vapor containing peracetic acid (also referred to as "peracetic acid vapor" hereinafter). The vapor generation unit 120 will be described later in detail. By circulating peracetic acid vapor in the decontamination system 10, the inside of the safety cabinet 200 is decontaminated by removing at least one of microorganisms and viruses present in the safety cabinet 200, for example.

The safety cabinet 200 is a box-shaped experimental facility for suppressing biohazards. An experimenter inserts his/her hands into a work area WA1, and conducts an experiment using a biological material, for example. The safety cabinet 200 includes a fan 205, HEPA (High Efficiency Particulate Air) filters 210 and 220, and a shutter 250. During the experiment, the fan 205 operates to generate an air flow, a clean air is discharged to the outside through the HEPA filter 210, and the clean air is supplied to the work area WA1 through the HEPA filter 220. The shutter 250 is configured to be openable and closable.

A communication hole 268 is formed in a ceiling portion of the safety cabinet 200, for example. The communication hole 268 is openable and closable. In the example shown in FIG. 1, the pipe 102 is inserted into the work area WA1 from below the shutter 250, and the pipe 101 is connected to the communication hole 268. The pipes 101 and 102 are connected to the pump 110. The pump 110 is configured to suction air from the pipe 101 side, and supply air to the pipe 102 side. Peracetic acid vapor generated from the vapor generation unit 120 is circulated in the decontamination system 10 through the operation of the pump 110.

The shutter 250 is closed during decontamination. There are cases where, even in a state in which the shutter 250 is closed, the inside of the safety cabinet 200 is not completely isolated from the outside, and a slight gap is formed therebetween. This gap is covered by a piece of cover tape during decontamination, for example.

Also, a BI (Biological Indicator) 230 is arranged in a space above the HEPA filter 210 prior to decontamination. After the decontamination is completed, a decontamination effect is checked based on the death status of the BI 230. The reason why the decontamination effect is checked based on the death status of the BI 230 is that the BI 230 is installed at a position farthest from the peracetic acid vapor introduction part and where peracetic acid vapor is most difficult to reach, on the downstream side of the HEPA filter 210. It is conceivable that, when the BI at the position where peracetic acid vapor is most difficult to reach died out, peracetic acid vapor has sufficiently permeated the inside of the safety cabinet 200. Note that there is no particular limitation on a BI bacterial count, and the BI bacterial count may be $10^3$ to $10^6$, for example.

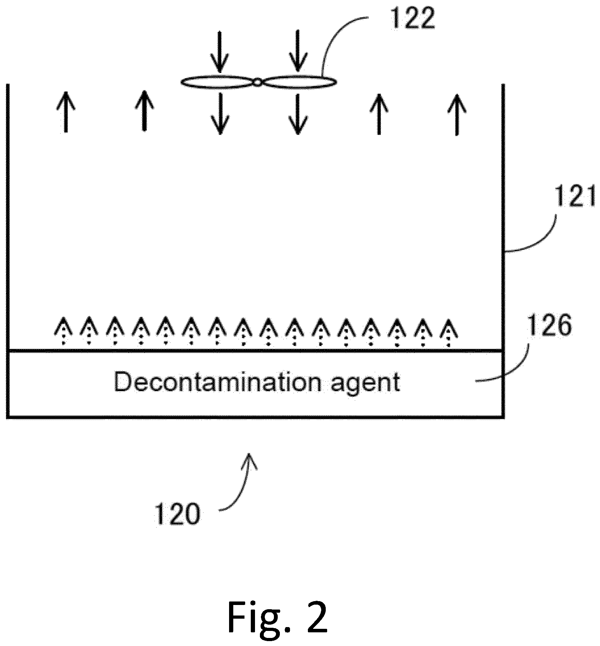

FIG. 2 is a diagram showing a schematic configuration of the vapor generation unit 120. As shown in FIG. 2, the vapor generation unit 120 includes a container 121 and a fan 122.

The container 121 is made of a material that is not corroded by a decontamination agent 126, such as a metal or resin, and is configured to accommodate the decontamination agent 126. The decontamination agent 126 is an aqueous solution containing peracetic acid. That is, the decontamination agent 126 is a peracetic acid formulation. The fan 122 is provided above the container 121.

The fan 122 is configured to rotate to generate an air flow flowing from the outside to the inside of the container 121 and to generate an air flow flowing from the inside to the outside of the container 121. The peracetic acid vapor generated through evaporation of the decontamination agent 126 is released to the outside of the container 121 by rotating the fan 122. Because only the peracetic acid vapor generated through evaporation of the decontamination agent 126 is released to the outside of the container 121, mist containing peracetic acid is not released in the vapor generation unit 120.

The inside of the safety cabinet 200 is decontaminated by peracetic acid vapor in the decontamination system 10 in this manner. The HEPA filters 210 and 220 are also decontaminated by decontaminating the inside of the safety cabinet 200. The reasons why decontamination is performed using peracetic acid vapor instead of mist containing peracetic acid in the decontamination system 10 will be described below. That is, the reasons why a vaporization method (vapor) is used instead of a mist method will be described.

If the HEPA filters 210 and 220 are decontaminated using mist containing peracetic acid, for example, the mist will be collected by the HEPA filters 210 and 220. As a result, the HEPA filters 210 and 220 may get wet with mist (droplets) adhering thereto, and a pressure loss may be increased.

Peracetic acid vapor is unlikely to be collected by the HEPA filters 210 and 220. Therefore, the problem of the mist method will not arise. For such reasons, a vaporization method is used in the decontamination system 10. An appropriate decontamination method in the case of using a vaporization method is adopted in the decontamination system 10.

Next, a method for generating vapor containing peracetic acid will be described. A method for heating a solution is known as a means for efficiently generating vapor from the solution. However, according to this method, a problem may arise that decomposition of peracetic acid progresses due to heating and a decontamination effect is lessened, and a problem may arise that heated vapor is cooled by a comparatively low-temperature part in the decontamination target space and dew condensation will occur. The occurrence of dew condensation causes problems such as corrosion of this part and staining due to evaporation residue.

According to the vapor generation unit 120, the decontamination agent 126 is not heated, and thus it is possible to efficiently generate peracetic acid vapor while suppressing decomposition of peracetic acid. Also, according to the decontamination system 10, the temperature of the decontamination agent 126 containing peracetic acid is maintained at about a temperature in the space in which the HEPA filters 210 and 220 to be decontaminated are disposed, thus reducing the occurrence of dew condensation in the space. A decontamination method used in the decontamination system 10 will be described below.

2. Decontamination method

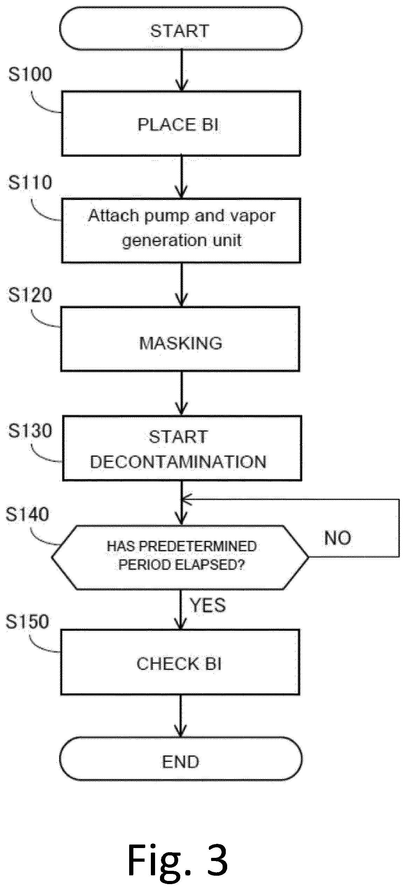

FIG. 3 is a flowchart showing an example of a procedure of the decontamination of the inside of the safety cabinet 200. Processes in this flowchart are performed by an operator.

Referring to FIG. 3, the operator places the BI 230 in the safety cabinet 200 (step S100). The operator places the vapor generation unit 120 in the safety cabinet 200, and connects the pump 110 to the safety cabinet 200 using the pipes 101 and 102 (step S110). The operator covers gaps in the safety cabinet 200 using a piece of cover tape or the like (step S120). The operator operates the pump 110 so as to start decontamination of the inside of the safety cabinet 200 (step S130).

The operator determines whether or not a predetermined period of time has passed (step S140). The operator waits until the predetermined period of time has passed (NO in step S140) . When the predetermined period of time has passed (YES in step S140), the operator checks a decontamination effect based on the death status of BI 230 (step S150). Decontamination of the inside of the safety cabinet 200 is completed by confirming the death of BI 230.

Note that the predetermined period of time in step S140 is the time required for the humidity in the safety cabinet 200 to be RH82% or more and RH99% or less, and the peracetic acid vapor load to be 2000 ppm or more and 12000 ppm or less. The predetermined period of time is determined through experiments in advance. Also, the peracetic acid vapor load is a value obtained by adding the concentration of peracetic acid in the peracetic acid vapor every minute from the start of decontamination to the completion of decontamination.

The inventor(s) of the present invention found through the later-described experiments that, when the average humidity in the safety cabinet 200 during decontamination is RH82% or more and the peracetic acid vapor load is 2000 ppm or more, the decontamination of the inside of the safety cabinet 200 succeeded. In the decontamination system 10, the average humidity in the safety cabinet 200 during decontamination is RH82% or more and the peracetic acid vapor load is 2000 ppm or more. Thus, according to the decontamination system 10, it is possible to appropriately decontaminate the inside of the safety cabinet 200.

Also, when the average humidity in the safety cabinet 200 during decontamination exceeds RH99%, dew condensation will occur in the safety cabinet 200. Dew condensation in the safety cabinet 200 causes corrosion and staining due to evaporation residue in the safety cabinet 200. Also, when the peracetic acid vapor load exceeds 12000 ppm, corrosion is likely to occur in the safety cabinet 200. The inventor(s) of the present invention found these facts. In the decontamination method 10, the average humidity is RH99% or less in the safety cabinet 200, and the peracetic acid vapor load is 12000 ppm or less. Thus, according to the decontamination system 10, it is possible to suppress corrosion in the safety cabinet 200. Note that, in the safety cabinet 200, the average humidity is preferably RH95% or less and the peracetic acid vapor load is more preferably 10000 ppm or less.

Also, the inventor(s) of the present invention found through the later-described experiment that decontamination of the inside of the safety cabinet 200 succeeds when the peracetic acid vapor load and the average humidity satisfy the following relationship. The inventor(s) found that, in the decontamination system 10 required of a high decontamination level in which a BI with a bacterial count of $10^6$ or more is used, for example, the peracetic acid vapor load and the average humidity need only satisfy the following relationship, in addition to the above-described conditions (RH82% or more, and the peracetic acid vapor load is 2000 ppm or more).

Therefore, according to the decontamination system 10, it is possible to appropriately decontaminate the inside of the safety cabinet 200.

$$y>-350x+33250$$

x: the average humidity
y: the peracetic acid vapor load

Further, in step S130 onward, the peracetic acid vapor is released for 50% or more of the time period from the start of the decontamination to the completion of the decontamination. Note that the time period during which peracetic acid vapor is released is preferably 70% or more and more preferably 90% or more of the time period from the start of the decontamination to the completion of the decontamination. Further, in step S130 onward, the longest time period of the time period during which the peracetic acid vapor is continuously released is 50% or more of the time period from the start of the decontamination to the completion of the decontamination. Note that the longest time period during which peracetic acid vapor is continuously released is preferably 70% or more and more preferably 90% or more of the time period from the start of the decontamination to the completion of the decontamination.

In the case of a mist method, for example, mist may be released intermittently in order to prevent the humidity in the safety cabinet 200 from increasing excessively. The threshold of the humidity for generating mist is often RH80% to RH92%. In the case of the mist method, the time period during which mist is released is often less than 30% of the time period from the start of the decontamination to the completion of the decontamination.

On the other hand, in the case of the vaporization method, because mist does not float therein, dew condensation is unlikely to occur.

In the decontamination system 10, it is possible to release vapor for a long time period of the time period from the start of the decontamination to the completion of the decontamination. The peracetic acid vapor can be released for 50% or more of the time period, for example. It is possible to release vapor for 100% of the time period from the start of the decontamination to the completion of the decontamination depending on a decontamination environment. Therefore, according to the decontamination system 10, because the vaporization method is adopted and the period of time in which the release of vapor is stopped is short, it is possible to inhibit the peracetic acid vapor from decomposing in the decontamination target space and inhibit the concentration of peracetic acid from greatly decreasing.

Further, in the decontamination system 10, the longest time period of the time period during which the peracetic acid vapor is continuously released is 50% or more of the time period from the start of the decontamination to the completion of the decontamination. Therefore, according to the decontamination system 10, because the vaporization method is adopted and vapor is continuously released for a long time period, it is possible to inhibit the concentration of peracetic acid in the safety cabinet 200 from greatly decreasing.

3. Features

As described above, in the decontamination method used in the decontamination system 10, the average humidity in the safety cabinet 200 during decontamination is RH82% or more and the peracetic acid vapor load is 2000 ppm or more. Therefore, according to this decontamination method, it is possible to appropriately decontaminate the inside of the safety cabinet 200.

4. Modification Examples

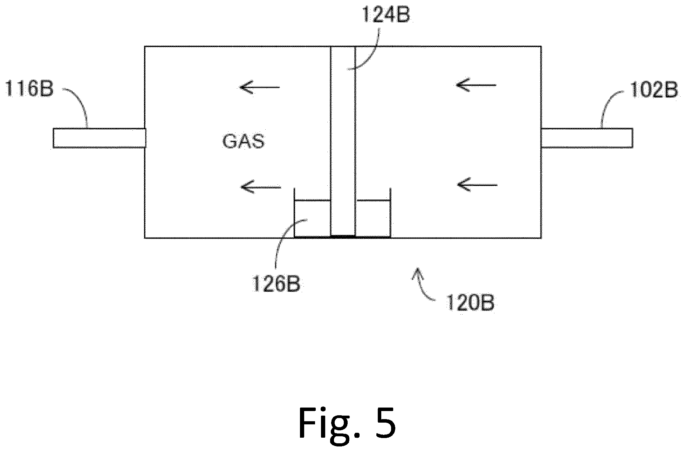

Although an embodiment of the present invention has been described above, the present invention is not limited to the above-described embodiment, and various modifications can be made without departing from the gist of the invention. The following describes modification examples.
<4-1>
Air filters disposed in the safety cabinet 200 are the HEPA filters 210 and 220 in the above embodiment. However, an air filter disposed in the safety cabinet 200 is not limited to these filters. An air filter to be decontaminated may be a medium efficiency penetration air filter or a ULPA filter, for example.
<4-2>
Also, the decontamination target is the safety cabinet 200 in the above embodiment. However, the decontamination target is not limited to this. The decontamination target may be any object as long as the inside thereof needs to be decontaminated, for example. Also, the decontamination target may be sealed or semi-sealed. That is, it is sufficient that the airtightness of the decontamination target is such that the concentration of the peracetic acid vapor does not decrease excessively due to leakage of the peracetic acid vapor. The decontamination target may be an isolator device, an incubator, a centrifuge, a pass box, a storage, an air conditioner, a clean bench, or a duct, for example.
<4-3>
Also, the vapor generation unit 120 is disposed in the safety cabinet 200 in the above embodiment. However, the configuration of the vapor generation unit 120 is not limited to this. The fan 122 need not be provided in the vapor generation unit 120, for example. Further, a water-absorbable vaporization facilitating member 124A may be provided. The water-absorbable vaporization facilitating member 124A may be formed by processing a sheet-shaped member such as a woven fabric, a knitted fabric, a non-woven fabric, or a film into a pleat shape or a corrugated shape, for example. In the water-absorbable vaporization facilitating member 124A, a woven fabric, a knitted fabric, a non-woven fabric, a film, or the like may contain a porous material such as silica gel or zeolite.

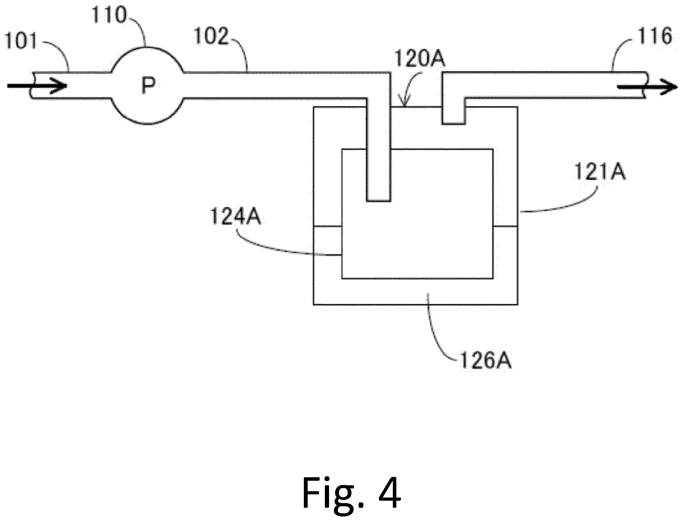

FIG. 4 is a diagram showing a schematic configuration of a vapor generation unit 120A according to a modification example. The vapor generation unit 120A may be adopted instead of the vapor generation unit 120. As shown in FIG. 4, pipes 101 and 102 are connected to a pump 110. The pump 110 is configured to suction air from the pipe 101 side, and supply air to the pipe 102 side.
The vapor generation unit 120A includes a container 121A, a water-absorbable vaporization facilitating member 124A, and a decontamination agent 126A. The container 121A is a tubular sealed container, for example. The water-absorbable vaporization facilitating member 124A and the decontamination agent 126A are accommodated in the container 121A. The decontamination agent 126A is a liquid chemical agent containing peracetic acid. The water-absorbable vaporization facilitating member 124A is made of a porous material, for example. The water-absorbable vaporization facilitating member 124A is immersed in the decontamination agent 126A. The water-absorbable vaporization facilitating member 124A sucks up the decontamination agent 126A in the container 121A through capillary action. That is, the water-absorbable vaporization facilitating member 124A is impregnated with the decontamination agent 126A.
Note that there is no particular limitation on the structure and members of the water-absorbable vaporization facilitating member 124A as long as the water-absorbable vaporization facilitating member 124A is made wet by the decontamination agent 126A, and the water-absorbable vaporization facilitating member 124A can efficiently gasify (vaporize) the decontamination agent 126A by allowing air to pass therethrough. The water-absorbable vaporization facilitating member 124A may be formed by processing a sheet-shaped member such as a woven fabric, a knitted fabric, a non-woven fabric, or a film into a pleat shape or a corrugated shape, for example. A woven fabric, a knitted fabric, a non-woven fabric, a film, or the like may contain a porous material such as silica gel or zeolite.
Vaporization of the decontamination agent 126A with which the water-absorbable vaporization facilitating member 124A is impregnated is facilitated in response to the supply of air from the pump 110 through the pipe 102. This generates peracetic acid vapor. The peracetic acid vapor is supplied into the safety cabinet 200 through the pipe 116. Note that the vapor generation unit 120A need not include the water-absorbable vaporization facilitating member 124A.
Also, the structure of the vapor generation unit 120 may be the structure shown in FIG. 5. That is, a water-absorbable vaporization facilitating member 124B sucks up a decontamination agent 126B in a vapor generation unit 120B through capillary action. The peracetic acid vapor (gas) flows to a pipe 116B side due to air introduced into the vapor generation unit 120B through the pipe 102B. The peracetic acid vapor is introduced into the safety cabinet 200 through a pipe 116B.

5. Experiments

5-1. Content of Experiments

The decontamination system 10 shown in FIG. 1 was prepared in this experiment. Class IIA safety cabinet SCV-1308EC II A2 manufactured by Hitachi Industrial Equipment Systems Co., Ltd. was used as the safety cabinet 200. The F12/D-3-6-1-1 sensor module: 00-1705 manufactured by ATI was used as a peracetic acid concentration meter for measuring the concentration of peracetic acid in the safety cabinet 200. A temperature and humidity logger LR5001 manufactured by Hioki E.E. Corporation was used as a thermometer and hygrometer for measuring the temperature and the humidity in the safety cabinet 200. MINNCARE manufactured by Cantel was used as the decontamination agent 126 (FIG. 2). HMV-091 $10^6$ and KCD-404 $10^4$ manufactured by MesaLabs were used as the BI 230. PM/100 manufactured by MesaLabs was used as a culture solution for the BI 230.

The pump 110 and the safety cabinet 200 were connected to each other using the pipes 101 and 102 such that air is sucked up from the downstream side of the HEPA filter 210, and air is supplied into the safety cabinet 200 from below the shutter 250. The inside of the safety cabinet 200 was sealed as much as possible by covering the safety cabinet 200. The vapor generation unit 120 was installed in the work area WA1 so as to generate peracetic acid vapor. Note that the fan 205 may have been operated during decontamination, but was not operated at this time. This is because, when the fan 205 operates and generates heat, the temperature of the air in the safety cabinet 200 will increase, and dew condensation may occur in a comparatively low temperature part inside the decontamination system 10.

The BI 230 was placed on the downstream side of the HEPA filter 210. After the decontamination is completed, the BI 230 was cultured.

MINNCARE (4.5% peracetic acid content) was diluted with pure water, and the resulting dilution was used as the decontamination agent 126. A diluted liquid with a dilution percentage of 1% to 25% was used and adjusted according to the concentration of peracetic acid vapor to be generated. An axial fan with a blade diameter of 11 cm was used as the fan 122.

During the decontamination, the temperature and the humidity in the safety cabinet 200, and the peracetic acid vapor concentration were recorded every minute. Positive/negative judgement was made after the BI 230 was cultured

5-2. Results of experiments

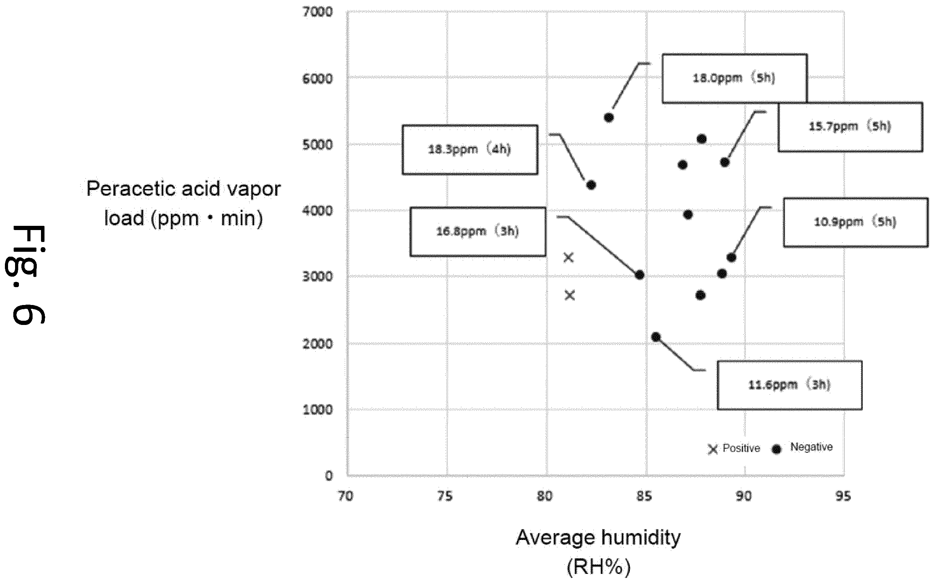

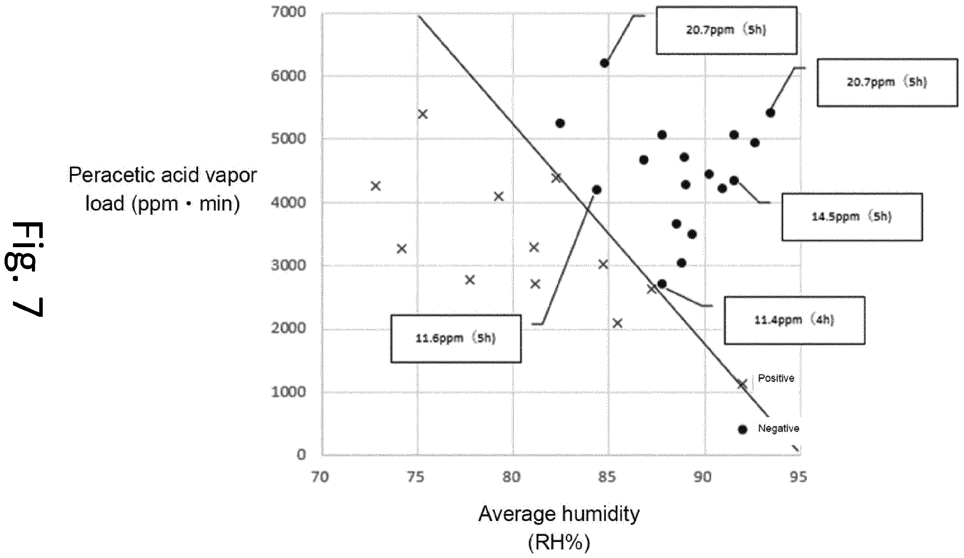

FIG. 6 is a diagram showing results in a case where a BI 230 with $10^4$ was used. FIG. 7 is a diagram showing results in a case where a BI 230 with $10^6$ was used. In FIGS. 6 and 7, the average humidity in the safety cabinet 200 (the average of humidity values obtained every minute from the start of the decontamination to the completion of the decontamination) is shown on the horizontal axis, and the peracetic acid vapor load in the safety cabinet 200 is shown on the vertical axis. Also, in FIGS. 6 and 7, with regard to representative examples, the peracetic acid concentration of the peracetic acid vapor generated by the vapor generation unit 120 is associated with the decontamination time period. "18.3 ppm (4 h)" indicates that the average peracetic acid concentration of the peracetic acid vapor generated by the vapor generation unit 120 is 18.3 ppm, and the time period from the start of the decontamination to the completion of the decontamination is 4 hours, for example.

Referring to FIGS. 6 and 7, the BI 230 did not die out unless the average humidity in the safety cabinet 200 was RH82% or more during decontamination (see a negative plot). Also, the death conditions of the BI 230 with $10^4$ were that the average humidity was RH82% or more, and the peracetic acid vapor load was 2000 ppm or more.

The death conditions of the BI 230 with $10^6$ were that the average humidity was RH82% or more, and the following relationship between the peracetic acid vapor load and the average humidity (the relationship indicated by a linear line in FIG. 7) was satisfied.

$$y > -350x + 33250$$

x: the average humidity
y: the peracetic acid vapor load

Also, the inside of the safety cabinet 200 was decontaminated in a state in which a copper plate was placed in the safety cabinet 200. In this case, when the peracetic acid vapor load exceeded 6000 ppm, the copper plate was slightly rusted. Therefore, it was found that the peracetic acid vapor load is preferably 12000 ppm or less, and more preferably 7000 ppm or less.

List of Reference Numerals

| 10 | Decontamination system. |
|---|---|
| 101, 102, 116 | Pipe |
| 110 | Pump |
| 120 | Vapor generation unit |
| 121, 121A | Container |
| 122, 205 | Fan |
| 124A | Water-absorbable vaporization facilitating member |
| 126, 126A | Decontamination agent |
| 200 | Safety cabinet |

| 268 | Communication hole |
| 210, 220 | HEPA filter |
| 230 | BI |
| 250 | Shutter |
| WA1 | Work area |

**Claims**

1. A decontamination method for decontaminating an inside of a decontamination target by removing at least one of a microorganism and a virus present in the decontamination target, the method comprising

   releasing vapor containing peracetic acid into the decontamination target, without releasing mist containing peracetic acid,
   wherein an average humidity inside the decontamination target during decontamination is RH82% or more, and
   a peracetic acid vapor load that is a value obtained by adding a concentration of peracetic acid in the vapor every minute from a start of the decontamination to a completion of the decontamination is 2000 ppm or more.

2. The decontamination method according to claim 1,

   wherein the average humidity is RH99% or less, and
   the peracetic acid vapor load is 12000 ppm or less.

3. The decontamination method according to claim 1 or 2,

   wherein the peracetic acid vapor load and the average humidity satisfy a relationship below,

   $$y > -350x + 33250$$

   x: the average humidity
   y: the peracetic acid vapor load.

4. The decontamination method according to any one of claims 1 to 3, wherein the vapor is released for 50% or more of a time period from the start of the decontamination to the completion of the decontamination.

5. The decontamination method according to any one of claims 1 to 4, wherein the longest time period of a time period during which the vapor is continuously released is 50% or more of a time period from the start of the decontamination to the completion of the decontamination.

Fig. 1

122

121

126

Decontamination agent

120

Fig. 2

START

S100 — PLACE BI

S110 — Attach pump and vapor generation unit

S120 — MASKING

S130 — START DECONTAMINATION

S140 — HAS PREDETERMINED PERIOD ELAPSED?   NO

YES

S150 — CHECK BI

END

# Fig. 3

Fig. 4

Fig. 5

Fig. 6

Fig. 7

## INTERNATIONAL SEARCH REPORT

| International application No. |
| --- |
| **PCT/JP2021/036527** |

**A. CLASSIFICATION OF SUBJECT MATTER**

*A61L 2/20*(2006.01)i; *A61L 9/01*(2006.01)i; *A61L 9/015*(2006.01)i; *A61L 101/36*(2006.01)n
FI:    A61L2/20; A61L9/01 H; A61L9/015; A61L101:36

According to International Patent Classification (IPC) or to both national classification and IPC

**B. FIELDS SEARCHED**

Minimum documentation searched (classification system followed by classification symbols)

A61L2/00-A61L2/28; A61L9/01; A61L9/015; A61L101/36

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2014-508579 A (FMC CORP.) 10 April 2014 (2014-04-10) paragraphs [0007]-[0016] | 1-5 |
| A | JP 2017-509408 A (STERIS INC.) 06 April 2017 (2017-04-06) paragraphs [0006]-[0046] | 1-5 |
| A | US 2019/0388574 A1 (PEROXYCHEM LLC) 26 December 2019 (2019-12-26) entire text | 1-5 |
| A | JP 2018-517539 A (MAR COR PURIFICATION INC.) 05 July 2018 (2018-07-05) entire text | 1-5 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **09 November 2021** | **16 November 2021** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
**Information on patent family members**

International application No.

**PCT/JP2021/036527**

| Patent document cited in search report | | | Publication date (day/month/year) | Patent family member(s) | | | Publication date (day/month/year) |
|---|---|---|---|---|---|---|---|
| JP | 2014-508579 | A | 10 April 2014 | US | 2012/0189494 | A1 | |
| | | | | paragraphs [0008]-[0016] | | | |
| | | | | WO | 2012/099959 | A2 | |
| | | | | AU | 2012207342 | A | |
| | | | | CA | 2823217 | A1 | |
| | | | | CN | 103298491 | A | |
| | | | | KR | 10-2014-0005190 | A | |
| | | | | RU | 2013129819 | A | |
| | | | | AU | 2016202043 | A | |
| | | | | CN | 108516154 | A | |
| | | | | BR | 112013016896 | A | |
| JP | 2017-509408 | A | 06 April 2017 | US | 2015/0265738 | A1 | |
| | | | | paragraphs [0007]-[0054] | | | |
| | | | | WO | 2015/143008 | A2 | |
| | | | | CA | 2941507 | A1 | |
| | | | | AU | 2015231409 | A | |
| | | | | CN | 106163578 | A | |
| | | | | MX | 2016011432 | A | |
| US | 2019/0388574 | A1 | 26 December 2019 | WO | 2019/246512 | A1 | |
| JP | 2018-517539 | A | 05 July 2018 | US | 2016/0346416 | A1 | |
| | | | | entire text | | | |
| | | | | WO | 2016/191377 | A1 | |
| | | | | AU | 2016268224 | A | |
| | | | | CA | 2987468 | A1 | |
| | | | | CN | 107708747 | A | |
| | | | | KR | 10-2018-0036651 | A | |

Form PCT/ISA/210 (patent family annex) (January 2015)

**EP 4 223 323 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018121826 A